Europäisches Patentamt

European Patent Office

Office européen des brevets

(11) Veröffentlichungsnummer: **0 338 976**

**A1**

## EUROPÄISCHE PATENTANMELDUNG

(21) Anmeldenummer: **89810217.3**

(22) Anmeldetag: **20.03.89**

(51) Int. Cl.⁴: **A 61 F 2/28**

(30) Priorität: **21.04.88 CH 1480/88**

(43) Veröffentlichungstag der Anmeldung:
**25.10.89 Patentblatt 89/43**

(84) Benannte Vertragsstaaten: **AT DE FR GB IT**

(71) Anmelder: **GEBRÜDER SULZER
AKTIENGESELLSCHAFT
Zürcherstrasse 9
CH-8401 Winterthur (CH)**

(72) Erfinder: **Frey, Otto, Dr.
Wallrütistrasse 56
CH-8400 Winterthur (CH)**

**Koch, Rudolf
Oberdorfstrasse 229
CH-8267 Berlingen (CH)**

(54) **Knochenimplantat aus Kunststoff.**

㊄ Eine aus einem mehrlagigen Drahtgewebe bestehende metallische Oberflächenstruktur (2) wird in einem Kunststoff-Implantat (1) ber zwei Lagen (6) des Gewebes verankert, die beispielsweise in dem zuvor durch Erwärmen "aufgeweichten" Kunststoff eingepresst werden.

Die Einbettung von zwei Verankerungslagen (6) in den Kunststoff 1) erhöht die Festigkeit der Verbindung zwischen Kunststoff (1) und Oberflächenstruktur (2).

Fig. 1

**EP 0 338 976 A1**

## Beschreibung

### Gebrüder Sulzer, Aktiengesellschaft, Winterthur/Schweiz

Knochenimplantat aus Kunststoff

Die Erfindung betrifft ein Knochenimplantat aus Kunststoff, dessen dem Knochen zugewandte Oberfläche mindestens teilweise mit einem mehrlagigen Drahtgewebe belegt ist, das mit mindestens einer Lage im Kunststoff verankert, und bei dem die Porengrössen der kunststofffreien Lagen nach aussen definiert und kontinuierlich zunehmen, wobei der Kunststoff von den kunststofffreien Lagen durch eine Trennschicht getrennt ist, deren Porengrösse kleiner ist als diejenige der im Kuntstoff verankerten Lage.

Ein Knochenimplantat der vorstehend genannten Art ist bekannt aus der EP-A-0 190 422; in der Praxis hat sich nun gezeigt, dass bei starken Belastungen die Verbindung zwischen Kunststoff und Drahtgewebe unter Umständen nicht ausreichend ist, da bei der bekannten Konstruktion das Drahtgewebe vom Kunststoff nur ungenügend umschlossen wird, so dass die Gefahr besteht, dass es ausbricht.

Mit der vorliegenden Erfindung soll daher die Haftung zwischen Kunststoff und Drahtgewebe verbessert werden; dieses wird dadurch erreicht, dass die Verankerung im Kunststoff über mindestens zwei Lagen des Drahtgewebes erfolgt, deren Porengrössen untereinander gleich sind.

Dabei hat es sich als vorteilhaft erwiesen, wenn - unabhängig von der Grösse der Verankerungsfläche von Drahtgewebe und Kunststoff -die Drahtstärke bei den Verankerungslagen mindestens 0,5 mm beträgt.

Mit zwei Verankerungslagen im Kunststoff entstehen - wie auch die Figuren der nachfolgenden Ausführungsbeispiele zeigen - zwischen den Drähten des Gewebes relativ dicke Kunststoff-"Brükken", die ein Ausbrechen des Drahtgewebes verhindern. Wie bereits bei der bekannten Konstruktion, ergibt sich nur den Aufbau der einzelnen Lagen aus Drahtgewebe eine geordnete Porenstruktur mit definierten Porengrössen. Um dabei durch alle Lagen möglichst "geradlinig" durchgehende Poren zu erhalten, ist es zweckmässig, wenn die Fadenrichtung mindestens einzelner benachbarter Lagen des mehrlagigen Gewebes von Lage zu Lage um 45° gedreht ist.

Besonders bei derartigen "geradlinigen" Poren - oder wenn beispielsweise der Kunststoff beim Verbinden mit dem Drahtnetz, wenn auch nur vorübergehend, zu einer relativ dünnflüssigen Flüssigkeit wird - besteht die Gefahr, dass auch ein engmaschiges Gitter als Trennschicht, durch die der Kunststoff von den kunststofffreien Lagen ferngehalten werden soll, nicht ausreichend ist. In diesem Fall hat es sich als zweckmässig erwiesen, wenn die Trennschicht aus einer geschlossenen Platte besteht.

Im folgenden wird die Erfindung anhand von Ausführungsbeispielen im Zusammenhang mit der Zeichnung näher erläutert.

Fig. 1 ist ein Querschnitt durch eine in einem Ausschnitt vergrössert dargestellte Oberfläche eines Kunststoff-Implantates, das beispielsweise zementfrei in einem Knochen verankert ist;

Fig. 2 gibt in gleicher Darstellung ein mehrlagiges Drahtgewebe wieder, bei dem die "Laufrichtung" der Drähte benachbarter Lagen um 45° gegeneinander verdreht sind.

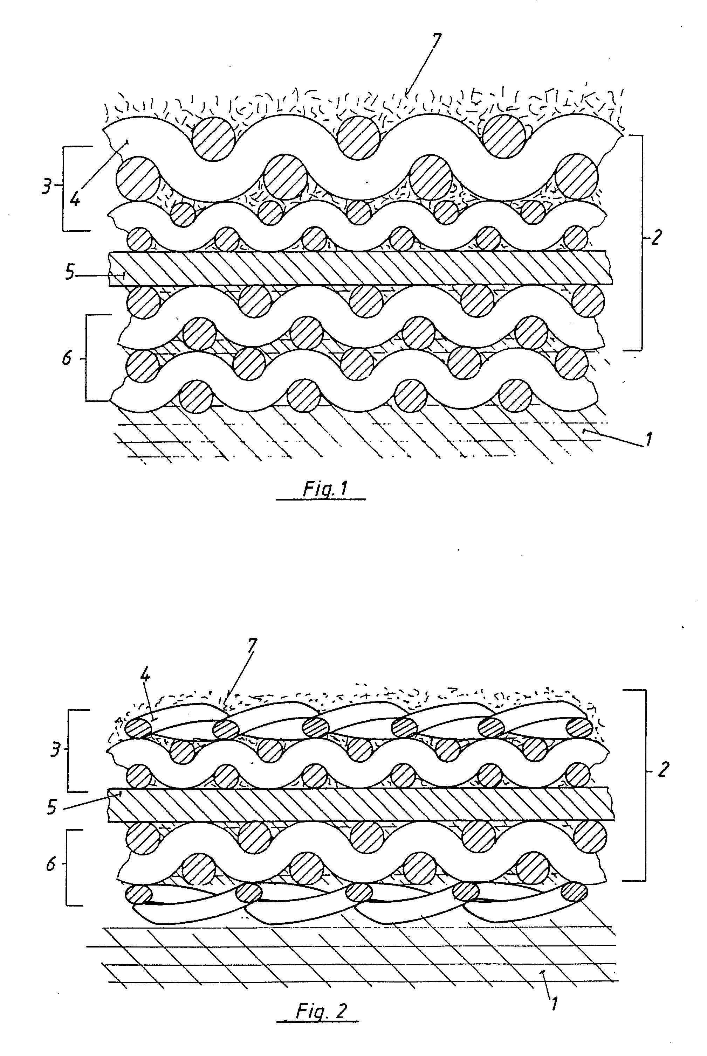

Fig. 1 ist beispielsweise ein Ausschnitt aus einer Polyäthylen-Hüftgelenkspfanne 1, deren Oberfläche mit einem Strukturelement 2 bedeckt ist, das im wesentlichen in einem mehrlagigen Drahtgewebe aus Titan oder einer Titanlegierung besteht. Die beiden äusseren in der Darstellung oberen Lagen 3 des Elementes sind zum Einwachsen von Knochengewebe 7 oder zur Aufnahme von Knochenzement bestimmt. Bei ihnen nimmt die Drahtstärke der Drähte 4, aus der sich die Maschenweite und damit ihre Porengrösse ergibt, in bekannter Weise von aussen nach innen ab.

Durch eine geschlossene Blechplatte 5, die Kontakte zwischen lebendem Gewebe 7 und Polyäthylen 1 absolut verhindert, sind die Lagen 3 von zwei weiteren Lagen 6 getrennt. Die Lagen 6 haben erfindungsgemäss untereinander gleiche Wandstärken und Porengrössen und sind beide in den Polyäthylenteil 1 eingepresst, um eine feste Verankerung der metallischen Oberflächenstruktur 2 im Polyäthylenteil 1 sicherzustellen.

Die Trennschicht 5 kann auch durch eine Lage eines engmaschigen Drahtgewebes, dessen Drahtstärke und Porengrösse kleiner als diejenigen der Verankerungslagen 6 und der inneren der beiden Lagen 3 sind, ersetzt sein.

Wie bereits erwähnt, kann die Durchgängigkeit der Poren verbessert werden, wenn innerhalb der Lagen 3 bzw. 6 benachbarte Drahtgewebe um 45° gegeneinander verdreht werden. Eine solche Anordnung ist in Fig. 2 dargestellt, die, abgesehen von der Drehung je einer Einwachs- und einer Verankerungslage, mit Fig. 1 übereinstimmt. Sind mehr als zwei Lagen vorhanden, so stimmt die Drehrichtung einer dritten Lage selbstverständlich wieder mit derjenigen der "ersten" Lage überein.

Die Drahtstärke der Drähte 4 betragen beispielsweise für die Lagen 3 zwischen 0,3 und 1 mm. Ist als Trennschicht ebenfalls eine Lage Gewebe vorgesehen, so werden dafür Drähte mit einem Durchmesser von 0,1 - 0,3 mm verwendet. Für die Verankerungslagen 6 haben sich unabhängig von dem das Drahtgewebe tragenden Kunststoffteil Drahtstärken von mindestens 0,5 mm besonders bewährt, mit denen sich Maschenweiten von etwa 1 mm ergeben.

Neben Titan oder seinen Legierungen können für die Drahtgewebe auch andere als gewebefreundlich bekannte Elemente, wie z.B. Tantal, Niob oder Zirkon, sowie Legierungen mit diesen Elementen verwendet werden. Weiterhin ist es möglich, zumindest die äusseren Lagen in bekannter Weise mit einer Beschichtung, beispielsweise aus Titannitrid oder einem anderen abriebsfesten und/oder das Einwachsen von Gewebe fördernden Werkstoff zu versehen.

Schliesslich kann es vorteilhaft sein, die Drahtgewebe durch Walzen vor dem Aufeinanderschichten der einzelnen Gitterlagen mit verbreiterten Auflageflächen zu versehen, um ihre gegenseitige Haftung zu verbessern, die beispielsweise mit Hilfe von örtlichen metallurgischen Bindungen erreicht wird. Derartige Bindungen lassen sich beispielsweise durch punktförmige Diffusionsschweissungen herstellen.

## Patentansprüche

1. Knochenimplantat aus Kunststoff, dessen dem Knochen zugewandte Oberfläche mindestens teilweise mit einem mehrlagigen Drahtgewebe belegt ist, das mit mindestens einer Lage im Kunststoff verankert, und bei dem die Porengrössen der kunstofffreien Lagen nach aussen definiert und kontinuierlich zunehmen, wobei der Kunststoff von den kunststofffreien Lagen durch eine Trennschicht getrennt ist, deren Porengrösse kleiner ist als diejenige der im Kunststoff verankerten Lage, **dadurch gekennzeichnet, dass** die Verankerung im Kunststoff (1) über mindestens zwei Lagen (16) des Drahtgewebes erfolgt, deren Porengrössen untereinander gleich sind.

2. Knochenimplantat nach Anspruch 1, **dadurch gekennzeichnet, dass** die Fadenrichtung mindestens einzelner, benachbarter Lagen (3 bzw. 6) des mehrlagigen Gewebes von Lage zu Lage um 45° gedreht ist.

3. Knochenimplantat nach Anspruch 1 oder 2, **dadurch gekennzeichnet, dass** die Trennschicht aus einer geschlossenen Platte (5) besteht.

4. Knochenimplantat nach einem der Ansprüche 1 bis 3, **dadurch gekennzeichnet, dass** die Drahtstärke der Verankerungslagen (6) mindestens 0,5 mm beträgt.

Fig. 1

Fig. 2

Europäisches
Patentamt

**EUROPÄISCHER RECHERCHENBERICHT**

Nummer der Anmeldung

EP 89 81 0217

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 539 209  (FACET ENTERPRISES) <br> * Figur 4; Seite 3, Zeilen 15-19; Seite 4, Zeilen 2-30 * | 1,3,4 | A 61 F   2/28 |
| Y | | 2 | |
| Y | EP-A-0 225 838  (TECHMEDICA INC.) <br> * Figur 2 * | 2 | |
| A | EP-A-0 190 422  (GEBRÜDER SULZER AG) <br> * Zusammenfassung * | 1 | |

RECHERCHIERTE
SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 18-07-1989 | ARGENTINI A. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsatze
E : älteres Patentdokument, das jedoch erst am oder
    nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes
    Dokument

EPO FORM 1503 03.82 (P0403)